# EUROPEAN PATENT APPLICATION

(11) **EP 4 311 537 A1**
(43) Date of publication of application: **31.01.2024**
(21) Application number: 22187086.8
(22) Date of filing: 26.07.2022
(51) Int. Cl.: A61K 8/99, A61Q 19/00, A61P 17/00, A61P 31/02, A61P 39/00

(54) **NOVEL SKIN CARE COMPOSITION FOR TREATING ATOPIC DERMATITIS**

(71) Applicant: Beiersdorf AG, 20245 Hamburg (DE)
(72) Inventor: HÜPEDEN, Jennifer, 20251 Hamburg (DE); FÖLSTER, Heike, 22149 Hamburg (DE); REUTER, Jörn Hendrik, 24558 Hamburg-Ulzburg (DE); GALLINAT, Stefan, 22880 Wedel (DE); AHLE, Charlotte, 13591 Berlin (DE)
(74) Representative: Uexküll & Stolberg

(57) **Abstract**

The present invention generally relates to the field of skin care. More particularly, the invention relates to a cosmetic or therapeutic skin care composition comprising at least one of the skin-health promoting *Staphylococcus* strains selected from the group of *Staphylococcus epidermidis* HAC26 and *Staphylococcus warneri* HAA333 or a culture supernatant of any of these. The invention also provides a method for treating or preventing atopic dermatitis and/or rosacea by applying the skin care composition of the invention to a skin area in need of treatment. The invention also relates to the use of a skin care composition of the invention for treating or preventing atopic dermatitis and/or rosacea.

## Description

The present invention generally relates to the field of skin care. More particularly, the invention relates to a cosmetic or therapeutic skin care composition comprising at least one of the skin-health promoting *Staphylococcus* strains selected from the group of *Staphylococcus epidermidis* HAC26 and *Staphylococcus warneri* HAA333 or a culture supernatant of any of these. The invention also provides a method for treating or preventing atopic dermatitis and/or rosacea by applying the skin care composition of the invention to a skin area in need of treatment. The invention also relates to the use of a skin care composition of the invention for treating or preventing atopic dermatitis and/or rosacea.

### BACKGROUND OF THE INVENTION

Atopic dermatitis, also known as atopic eczema or neurodermatitis, is a complex, multifactorial, chronic relapsing inflammatory condition of the skin. It results from an interaction of genetic and environmental factors as well as immunological and skin structural disorders. The Incidence of atopic dermatitis is dramatically increasing in industrialized nations. Today, about 15-20% of children and 1-3% of adults worldwide are affected by the disease, thereby creating a significant burden for heath care systems.

One of the hallmarks of atopic dermatitis is the dysfunction of the skin barrier. While an intact skin barrier protects the organism from both water loss and prevents the penetration of harmful substances such as irritants, allergens and microorganisms, increased water loss is observed in the extremely dry skin that accompanies atopic dermatitis. It was found that in patients suffering from atopic dermatitis, the permeability barrier of the skin becomes leaky in the course of the disease, thereby allowing allergens and microorganisms to permeate it and enter the skin, thereby causing infection (Silverberg & Silverberg, 2015).

The barrier permeability function of the skin is mainly located in the epidermis. The epidermis of the skin consists of several layers that act as barriers to prevent water loss and to protect the body from such foreign substances as microbes and allergens. It was found that Filaggrin (filament aggregating protein, FLG), a protein that binds to keratin fibers in epithelial cells, plays an important role in skin barrier function. In the epidermis, the FLG protein is associated with other proteins and lipids, thereby forming a strong barrier that prevent microbes and allergens from entering the deeper skin layers and minimizes transepidermal water loss (Sandilands et al. 2009). Mutations in the gene encoding that protein turned out to be a major risk factor for atopic dermatitis (Sicherer & Leung, 2009; Ring et al., 2012; Brown & Irvine, 2008; Scharschmidt TC, et al. 2009).

It was speculated that the down-regulation of FLG could render the skin more susceptible to immune dysregulation which further deteriorates the conditions of the skin and exacerbates atopic dermatitis. The immune dysregulation that occurs during atopic dermatitis is characterized by a complex of alterations involving interactions between IgE-bearing antigen-presenting cells, T-cell activation, mast-cell degranulation, keratinocytes, eosinophils, and a combination of immediate and cellular immune responses. Inflammatory dendritic epidermal cells constitute a distinct dendritic cells population that is mainly found in atopic dermatitis and could induce the Th2/Th1 isotopic switch contributing to atopic dermatitis chronic phase (Miraglia del Giudice et al. 2006).

In addition, it has been reported that the microbiome of patients afflicted with atopic dermatitis significantly differs from that of healthy subjects. It appears that part of the resident bacterial commensal flora is displaced by transient pathogenic species, in particular Staphylococcus aureus species. These species can colonize the skin and contribute to the deterioration of skin appearance. For example, *S. aureus* species, if present in excessive amount, can lead to skin dryness, eczematous or oily skin, all of which promotes the development of atopic dermatitis. On skin that is already affected by atopic dermatitis, S. *aureus* species can contribute significantly to the aggravation of skin inflammation and undesired symptoms like itching.

During the last decades, topical corticosteroids and calcineurin inhibitors have been the mainstay of treatment for atopic dermatitis. Strong corticosteroids are used only at the beginning of treatment in severe disease because they promote decreased formation of epidermal lipids and atrophy of the skin. Calcineurin inhibitors such as pimecrolimus, on the other hand, help to restore the skin barrier. In the case of excessive colonization of the skin with bacteria or fungi, infection-inhibiting ointments are used. In the case of excessive colonization by S. aureus, antibiotics like fusidic acid are regularly used. However, fusidic acid can cause severe side effects, such as liver damage and jaundice. In addition, pathogenic strains have developed a resistance to fusidic acid (Dobie & Gray, 2004).

In light of the above, there is a considerable need for new treatment approaches that are not associated with the above drawbacks. In particular, the new treatment should be highly effective, well tolerable, not susceptible to resistance and free of side effects. The present invention is based on the finding that naturally occurring *Staphylococcus* strains can be isolated that exert a high anti-bacterial against *S. aureus* strains. Accordingly, these strains are particularly useful as an active agent for preventing or reducing the number *of S. aureus* cells on the skin of a subject, preferably a human subject. When applied to the skin in the form of a skin care composition, the *Staphylococcus* strains of the invention can establish themselves in the skin flora and can effectively and eradicate or reduce the abundance *of S. aureus* strains.

### SUMMARY OF THE INVENTION

In a first aspect, the invention relates to a skin care composition for topical administration to the skin comprising
(a) *Staphylococcus epidermidis* strain HAC26, deposited at the DSMZ under accession number DSM 34117, and/or
(b) *Staphylococcus warneri* strain HAA333, deposited at the DSMZ under accession number DSM 34118
or a culture supernatant obtained from any of these strains. If culture supernatant is used, said supernatant is preferably sterile-filtered before adding it to the skin care composition.

In one embodiment, the *S. epidermidis* strain is present in the composition in lyophilized or spray-dried form. The *S. epidermidis* strain preferably is present in the composition in an amount of 10⁴-10¹¹ CFU/ml, preferably 10⁷-10¹⁰ CFU/ml. The *S. epidermidis* strain may be present in the composition in an amount of at least 0.5% (w/v) of the skin care composition.

In another embodiment, the *S. warneri* strain is present in the composition in lyophilized or spray-dried form. The *S. warneri* strain preferably is present in the composition in an amount of 10⁴-10¹¹ CFU/ml, preferably 10⁷-10¹⁰ CFU/ml. The *S. warneri* strain may be present in the composition in an amount of at least 0.5% (w/v) of the skin care composition.

In a particularly preferred embodiment, the skin care composition of the present invention comprises
(a) *S. epidermidis* strain HAC26 in an amount of 10⁴-10¹¹ CFU/ml, and preferably in an amount of 10⁷-10¹⁰ CFU/ml, and/or
(b) *S. warneri* strain HAA333 in an amount of 10⁴-10¹¹ CFU/ml, and preferably in an amount of 10⁷-10¹⁰ CFU/ml.

Within said composition, the two *Staphylococcus* strains are preferably present in approximately equal concentrations, i.e. in concentrations that differ from each other by not more than 5 % (w/v).

In one embodiment, the skin care composition further comprises one or more excipients, wherein said one or more excipients preferably comprise an emollient, a filler, a thickener, a solubilizer, an antioxidant, a preservative, a pH adjuster, a binder, a buffering agent, a colorant, a humectants, a exfoliating agent, a preservative, a plant extract, an essential oils, or a fragrance.

The skin care composition is preferably formulated as a gel, cream, ointment or lotion.

In a second aspect, the invention relates to a skin care composition for use in a method of treating atopic dermatitis and/or rosacea in a subject, reducing the number *of S. aureus* cells on the skin of a subject, and/or treating an infection of the skin of a subj ect.

In a third aspect, the invention relates to *S. epidermidis* strain HAC26, deposited at the DSMZ under accession number DSM 34117 or a culture supernatant obtained from said strain and S. *warneri* strain HAA333, deposited at the DSMZ under accession number DSM 34118 or a culture supernatant obtained from said strain.

### DESCRIPTION OF THE FIGURE

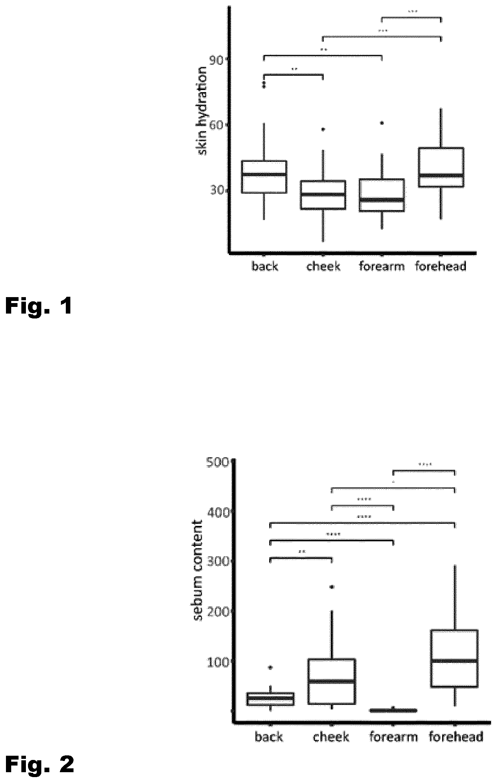

Figure 1 shows the results from the skin hydration measurement at the locations from which coagulase-negative staphylococci were isolated. Skin hydration data are shown for back, cheek, forearm, and forehead skin of the test subjects (^{∗}p ≤ 0.05, ^{∗∗}p ≤ 0.01, ^{∗∗∗}p ≤ 0.001, ^{∗∗∗∗}p ≤ 0.0001, unpaired Wilcoxon test).
Figure 2 shows the results from the sebum content measurement at the locations from which coagulase-negative staphylococci were isolated. Sebum content data are shown for back, cheek, forearm, and forehead skin of the test subjects (^{∗}p ≤ 0.05, ^{∗∗}p ≤ 0.01, ^{∗∗∗}p ≤ 0.001, ^{∗∗∗∗}p ≤ 0.0001, unpaired Wilcoxon test).

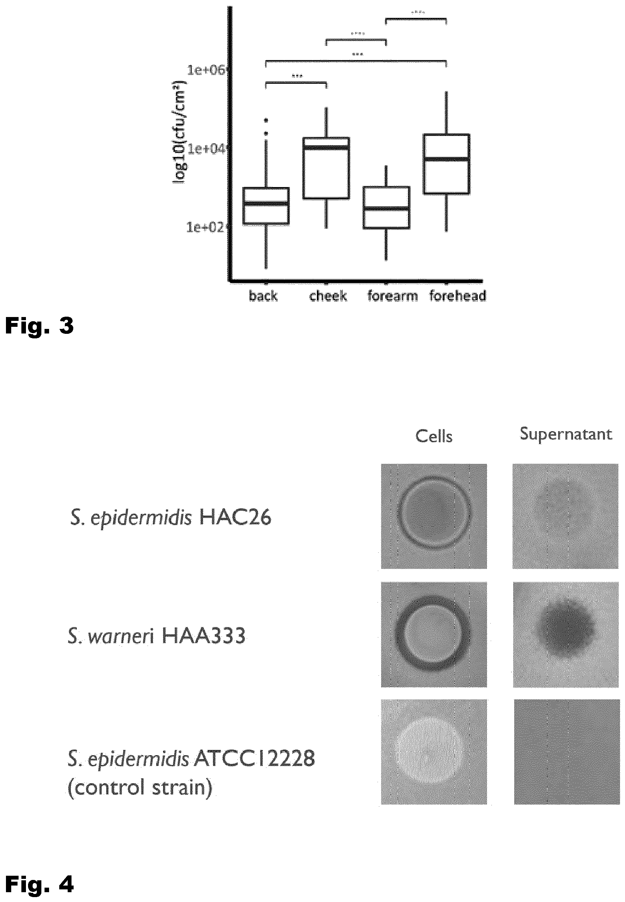

Figure 3 shows the results from the cell count measurement at the locations from which coagulase-negative staphylococci were isolated. Cell count results in CFUs per cm² skin surface are shown for back, cheek, forearm, and forehead skin of the test subjects (^{∗}p ≤ 0.05, ^{∗∗}p ≤ 0.01, ^{∗∗∗}p ≤ 0.001, ^{∗∗∗∗}p ≤ 0.0001, unpaired Wilcoxon test).
Figure 4 shows the antimicrobial properties of *S. epidermidis* strain HAC26 and *S. warneri* strain HAA333 against *S. aureus DSM799,* as determined in the lawn plate test.

### DETAILED DESCRIPTION

The present invention provides novel *Staphylococcus* strains that were isolated from natural environments and show a significant activity against *Staphylococcus aureus* strains, and in particular *S. aureus* strains that occur on skin that is afflicted with atopic dermatitis and rosacea. In particular, the present invention provides the *S. epidermidis* strain HAC26 which was deposited at the DSMZ on December 14, 2021 and received the accession number DSM 34117 and *S. warneri* strain HAA333 which was deposited at the DSMZ on December 14, 2021 and received the accession number DSM 34118. The invention also relates to culture supernatants of any of theses strains.

Owing to their ability to control and reduce the abundance of *S. aureus* strains on human skin, these strains or culture supernatants obtained from these strains can be used as active ingredients in cosmetic and pharmaceutical skin care compositions which are topically applied to skin comprising an excessive number of one or more *S. aureus* strains. The activity of the strains of the invention is based on the production and secretion of antibacterial compounds that are active against *S. aureus* strains. As these antibacterial compounds are secreted into the surrounding medium, it is also possible to use culture supernatants which are obtained from said strain. As used herein, culture supernatants are fractions of the cell culture medium that had been used for culturing the cells and in which compounds that were secreted from the cultured cells were accumulated.

In the context of the present invention, *S. epidermidis* strain HAC26 or *S. warneri* strain HAA333 is considered as exerting activity against a S. *aureus* strain, if the strain is active in the following lawn assay. A culture of *S. epidermidis* strain HAC26 or *S. warneri* strain HAA333 is prepared and incubated overnight at 37°C in CASO broth until an OD₆₀₀ of 8.0 is reached. Subsequently, 15 µl of the overnight culture are pipetted to a lawn plate of the respective *S. aureus* strain which comprises at least 4×10⁵ CFU/plate. The *S. epidermidis* strain is active against the *S. aureus* strain, if a zone of inhibition appears around the site of application after incubation of the plate for 24 hours at 37°C.

Therefore, the invention also provides skin care composition for topical administration to the skin comprising
(a) *Staphylococcus epidermidis* strain HAC26, deposited at the DSMZ under accession number DSM 34117, and/or
(b) *Staphylococcus warneri* strain HAA333, deposited at the DSMZ under accession number DSM 34118, and/or
(c) culture supernatants obtained from any of the above strains.

The S. *epidermidis* strain and/or the *S. warneri* strain may be present in the composition of the invention in lyophilized or spray-dried form. This means that viable bacteria have been subjected to a drying process that maintains their viability, but reduces their metabolic processes to minimum. In lyophilized or spray-dried form, the bacteria can be stored for months or even years. Once they are applied to the skin, such as the human skin, the metabolism of the bacteria is reactivated such that they resume growth. They propagate on the skin surface and displace pathogenic bacterial strains, thereby recovering a diverse, healthy and balanced skin microbiome.

In one embodiment, the *S. epidermidis* strain and/or said *S. warneri* strain are present in spray-dried form. The principle of spray drying is based on the dispersion of a solution into fine droplets which are introduced into a flow of hot air. The solvent evaporates from the substrate droplets so that dry product clusters remain. Standard spray drying devices can be used, such as the Mini Spray Dryer B-290 from Büchi Labortechnik GmbH (Essen, Germany) or the Mobile Minor^{™} Spray Dryer from GEA (Berlin, Germany). In another embodiment, the *S. epidermidis* strain and/or said *S. warneri* strain are present in freeze-dried or lyophilized form. Freeze drying or lyophilization is a process which includes freezing the product, reducing the pressure and adding heat to allow the frozen water in the material to sublimate. Various methods can be applied for freezing the product. For example, freezing can be achieved by using a standard freezer or a chilled bath. Cooling the product below its triple point ensures that sublimation will occur upon heating. To prevent the formation of large crystals that may damage the structure of the product to be dried, freezing is done rapidly. About 95% of the water in the product is removed when the frozen water sublimates. Most materials can be dried to 1-5% residual moisture. Standard freeze drying devices can be used, such as the Lyovac^{™} devices from GEA (Berlin, Germany), the Gamma 2-20 Freeze dryer LCM-1 from Christ (Osterode am Harz, Germany), or the Christ Martin^{™} Alpha 1-2 Lyophilisator from Fisher Scientific GmbH (Schwerte, Germany).

It is preferred that the *S. epidermidis* strain HAC26 is present in the composition in an amount of 1.0 × 10⁴-1.0 × 10¹¹ colony forming units (CFU) per ml, more preferably 1.0 × 10⁵-1.0 × 10¹⁰ CFU/ml, and even more preferably 1.0 × 10⁷-1.0 × 10¹⁰ CFU/ml, or 1.0 × 10⁸-1.0 × 10⁹ CFU/ml. For example, the *S. epidermidis* strain HAC26 may be present in an amount of at least 1.0 × 10⁵ CFU/ml, preferably at least 1.0 × 10⁶ CFU/ml, more preferably at least 1.0 × 10⁷ CFU/ml, such as at least 1.0 × 10⁸ CFU/ml, at least 1.0 × 10⁹ CFU/ml, or at least 1.0 × 10¹⁰ CFU/ml of the skin care composition. It is particularly preferred that the *S. epidermidis* strain HAC26 is present in an amount of at least 1.0 × 10¹⁰ CFU/ml, 2.0 × 10¹⁰ CFU/ml, 3.0 × 10¹⁰ CFU/ml, 4.0 × 10¹⁰ CFU/ml, 5.0 × 10¹⁰ CFU/ml, 6.0 × 10¹⁰ CFU/ml, 7.0 × 10¹⁰ CFU/ml, 8.0 × 10¹⁰ CFU/ml, or 9.0 × 10¹⁰ CFU/ml of the skin care composition.

Stated differently, the skin care composition of the invention comprises an amount of at least 0.5% (w/v) of the *S. epidermidis* strain HAC26, based on the overall volume of the skin care composition. More preferably, the skin care composition of the invention comprises an amount of at least 0.75% (w/v), at least 1.0% (w/v), at least 1.25% (w/v), at least 1.5% (w/v), at least 1.75% (w/v), at least 2.0% (w/v), at least 2.25% (w/v), or at least 2.5% (w/v) of the of the S. *epidermidis* strain HAC26, based on the overall volume of the skin care composition.

Similarly, it is preferred that the *S. warneri* strain HAA333 is present in the composition in an amount of 1.0 × 10⁴-1.0 × 10¹¹ CFU per ml, more preferably 1.0 × 10⁵-1.0 × 10¹⁰ CFU/ml, and even more preferably 1.0 × 10⁷-1.0 × 10¹⁰ CFU/ml, or 1.0 × 10⁸-1.0 × 10⁹ CFU/ml. For example, the *S. warneri* strain HAA333 may be present in an amount of at least 1.0 × 10⁵ CFU/ml, preferably at least 1.0 × 10⁶ CFU/ml, more preferably at least 1.0 × 10⁷ CFU/ml, such as at least 1.0 × 10⁸ CFU/ml, at least 1.0 × 10⁹ CFU/ml, or at least 1.0 × 10¹⁰ CFU/ml of the skin care composition. It is particularly preferred that the *S. warneri* strain HAA333 is present in an amount of at least 1.0 × 10¹⁰ CFU/ml, 2.0 × 10¹⁰ CFU/ml, 3.0 × 10¹⁰ CFU/ml, 4.0 × 10¹⁰ CFU/ml, 5.0 × 10¹⁰ CFU/ml, 6.0 × 10¹⁰ CFU/ml, 7.0 × 10¹⁰ CFU/ml, 8.0 × 10¹⁰ CFU/ml, or 9.0 × 10¹⁰ CFU/ml of the skin care composition.

Stated differently, the skin care composition of the invention comprises an amount of at least 0.5% (w/v) of the *S. warneri* strain HAA333, based on the overall volume of the skin care composition. More preferably, the skin care composition of the invention comprises an amount of at least 0.75% (w/v), at least 1.0% (w/v), at least 1.25% (w/v), at least 1.5% (w/v), at least 1.75% (w/v), at least 2.0% (w/v), at least 2.25% (w/v), or at least 2.5% (w/v) of the of the *S. warneri* strain HAA333, based on the overall volume of the skin care composition.

When both the *S. epidermidis* strain HAC26 and the *S. warneri* strain HAA333 are used in combination in the same skin care composition, each of the two strains is preferably present in the composition in an amount of 1.0 × 10⁴-1.0 × 10¹¹ CFU/ml, more preferably 1.0 × 10⁵-1.0 × 10¹⁰ CFU/ml, and even more preferably 1.0 × 10⁷-1.0 × 10¹⁰ CFU/ml, or 1.0 × 10⁸-1.0 × 10⁹ CFU/ml. For example, each of the strains may be present in an amount of at least 1.0 × 10⁵ CFU/ml, preferably at least 1.0 × 10⁶ CFU/ml, more preferably at least 1.0 × 10⁷ CFU/ml, such as at least 1.0 × 10⁸ CFU/ml, at least 1.0 × 10⁹ CFU/ml, or at least 1.0 × 10¹⁰ CFU/ml of the skin care composition. It is particularly preferred that each of the strains is present in an amount of at least 1.0 × 10¹⁰ CFU/ml, 2.0 × 10¹⁰ CFU/ml, 3.0 × 10¹⁰ CFU/ml, 4.0 × 10¹⁰ CFU/ml, 5.0 × 10¹⁰ CFU/ml, 6.0 × 10¹⁰ CFU/ml, 7.0 × 10¹⁰ CFU/ml, 8.0 × 10¹⁰ CFU/ml, or 9.0 × 10¹⁰ CFU/ml of the skin care composition.

In some embodiments, the overall amount of lyophilized or spray-dried bacteria in the composition is 1.0 × 10⁴-1.0 × 10¹¹ CFU/ml, more preferably 1.0 × 10⁵-1.0 × 10¹⁰ CFU/ml, and even more preferably 1.0 × 10⁷-1.0 × 10¹⁰ CFU/ml, or 1.0 × 10⁸-1.0 × 10⁹ CFU/ml. For example, the bacteria may be collectively present in the composition in an amount of at least 1.0 × 10⁵ CFU/ml, preferably at least 1.0 × 10⁶ CFU/ml, more preferably at least 1.0 × 10⁷ CFU/ml, such as at least 1.0 × 10⁸ CFU/ml, at least 1.0 × 10⁹ CFU/ml, or at least 1.0 × 10¹⁰ CFU/ml of the skin care composition. It is particularly preferred that the bacteria are collectively present in the composition in an amount of at least 1.0 × 10¹⁰ CFU/ml, 2.0 × 10¹⁰ CFU/ml, 3.0 × 10¹⁰ CFU/ml, 4.0 × 10¹⁰ CFU/ml, 5.0 × 10¹⁰ CFU/ml, 6.0 × 10¹⁰ CFU/ml, 7.0 × 10¹⁰ CFU/ml, 8.0 × 10¹⁰ CFU/ml, or 9.0 × 10¹⁰ CFU/ml of the skin care composition. One of ordinary skill in the art will be readily able to determine the amount of bacteria in a lyophilized or spray-dried composition.

When both the *S. epidermidis* strain HAC26 and the *S. warneri* strain HAA333 are used in combination in the same skin care composition, it is particularly preferred that both strains are present in the composition are present in approximately equal concentrations, i.e. cell numbers that differ from each other in not more than 10%.

In one embodiment, the culture supernatant of a culture of one or more of the strains of the invention is added to the skin care composition of the invention. Preferably, the culture supernatant is sterile-filtered before adding it to the skin care composition. For example, the culture supernatant can be sterile-filtered by centrifugation of an overnight culture for 10 min at 8000 g followed by filtration of the supernatant with a filter having a pore size of 0.2 µm.

The skin care composition of the invention is formulated for topical application which means that the composition is provided in a form that allows the consumer to dispense the composition after application to the skin. The skin care composition of the invention can be formulated as a liquid, ointment, cream, scrub, lotion, paste, gel, hydrogel, foam, or powder. It is however preferred that is formulated as a gel, cream, ointment or lotion. The skin care composition may be formulated be packaged into a pump dispenser which allows spraying the liquid onto the skin areas to be treated. The skin care composition of the invention can also be incorporated into a patch which is applied to the skin.

The composition of the invention may optionally comprise one or more excipients that are commonly used in cosmetic or pharmaceutical skin care products. Such excipients are described in detail, for example, in the International Cosmetic Ingredient Dictionary and Handbook, 16th ed. (2016). For example, the one or more excipients may comprise an emollient, an exfoliating agent, a humectant, a pH adjuster, a thickener, a solubilizer, an antioxidant, a preservative, a filler, a binder, a buffering agent, a colorant, an essential oil, a pigment, a sunscreen, an antiseptic, a chelating agent, a vitamin, panthenol, ubiquinone Q10, hyaluronic acid, or any combinations thereof.

For example, the skin care composition of the invention may comprise an emollient. As used herein, an emollient is a compound that moisturizes and/or softens the skin. Emollients normally reduce the roughness, cracking and/or irritation of the skin by penetrating into the deeper layers of the skin. Suitable emollients for the skin care composition of the present invention include, but are not limited to, olive oil, palm oil, soybean oil, sesame seed oil, rapeseed oil, evening primrose oil, sunflower seed oil, avocado oil, olive oil, coconut oil, castor oil, safflower seed oil, myristyl lactate, isopropyl myristate, polyethylene glycol, isopropyl palmitate, isopropyl stearate, isobutyl palmitate, isocetyl stearate, or cetyl alcohol. Preferably, the emollient is present in the skin care composition of the invention in an amount of 0.05% (w/w) to 10% (w/w), preferably 0.1 to 10.0% (w/w), and more preferably 1.0 to 10.0% (w/w), based on the total weight of the composition. In another embodiment, the emollient is present in the skin care composition of the invention in an amount of 0.05% (w/w) to 5.0% (w/w), preferably 0.1 to 5.0% (w/w), and more preferably 1.0 to 5.0% (w/w), based on the total weight of the composition. The skin care composition of the invention may also comprise more than one emollient. In this case the above amounts preferably refer to the overall amount of emollients in the composition.

The skin care composition of the invention may also comprise an exfoliating compound. Exfoliating compounds are solids that provide for a granular texture of the composition. When rubbed onto the skin, these compounds eliminate old skin cells and enhance skin renewal. Exfoliating compounds which are useful in the compositions of the present invention include, but are not limited to, urea, alpha-hydroxy acids and beta-hydroxy acids, and their esters, anhydrides, and salts. Suitable hydroxy acids include, for example, urea, glycolic acid, lactic acid, malic acid, mandelic acid, tartaric acid, citric acid, 2-hydroxyalkanoic acid, salicylic acid, and derivatives thereof. The use of urea is particularly preferred, because it also has an additional water-binding effect and enhances the absorption of other cosmetic ingredients. Preferably, the exfoliating compound is present in the skin care composition of the invention in an amount of 0.05% (w/w) to 10% (w/w), preferably 0.1 to 10.0% (w/w), and more preferably 1.0 to 10.0% (w/w), based on the total weight of the composition. In another embodiment, the exfoliating compound is present in the skin care composition of the invention in an amount of 0.05% (w/w) to 5.0% (w/w), preferably 0.1 to 5.0% (w/w), and more preferably 1.0 to 5.0% (w/w), based on the total weight of the composition. The skin care composition of the invention may also comprise more than one exfoliating compound. In this case the above amounts preferably refer to the overall amount of exfoliating compounds in the composition.

The skin care composition of the present invention may also comprise a humectant for improving skin hydration. Suitable humectants for use in the composition of the present invention include, but are not limited to, glycerine, polyethylene glycol ethers of glycerine, amino acids, such as proline and arginine, sugar and sugar alcohols, such as glucose, mannose, trehalose, and polyglycerol sorbitol, 1,3-butylene glycol, propylene glycol, diglycerol, glycerol monopropoxylate, glycogen, sodium hyaluronate, sodium poly-aspartate, sodium polyglutamate, sorbeth 20, sorbeth 6, and hydrogenated starch hydrolysates. Preferably, the humectant is present in the skin care composition of the invention in an amount of 0.05% (w/w) to 10% (w/w), preferably 0.1 to 10.0% (w/w), and more preferably 1.0 to 10.0% (w/w), based on the total weight of the composition. In another embodiment, the humectant is present in the skin care composition of the invention in an amount of 0.05% (w/w) to 5.0% (w/w), preferably 0.1 to 5.0% (w/w), and more preferably 1.0 to 5.0% (w/w), based on the total weight of the composition. The skin care composition of the invention may also comprise more than one humectant. In this case the above amounts preferably refer to the overall amount of humectants in the composition.

In one preferred embodiment, the skin care composition of the present invention comprises a compound that serves as a pH adjuster. Since the composition of the invention is used on the human skin, it will normally have a slightly acidic pH to make it more compatible with the acidic environment of the skin. The composition may have a pH in the range from about 2.5 to about 6.5, preferably from about 4.0 to about 6.0, and more preferably from about 5.0 to about 6.0 or from about 5.5 to about 6.0. The acidic pH can be achieved by adding an acid to the skin care composition of the invention, e.g. a carboxylic acid, such as an alpha hydroxy acid. The nature of the acid that can be used in the composition of the invention is not particularly limited. Suitable acids include formic acid, acetic acid, propionic acid, butyric acid, valeric acid, caproic acid, enanthic acid, caprylic acid, and the like. In a particular preferred embodiment, the composition comprises lactic acid as a pH adjuster. For application to the human skin, lactic acid is particularly useful, as it is also secreted by the skin flora to form the protective acidic milieu on the human skin surface. Preferably, the pH adjuster is present in the skin care composition of the invention in an amount of 0.05% (w/w) to 10% (w/w), preferably 0.1 to 10.0% (w/w), and more preferably 1.0 to 10.0% (w/w), based on the total weight of the composition. In another embodiment, the pH adjuster is present in the skin care composition of the invention in an amount of 0.05% (w/w) to 5.0% (w/w), preferably 0.1 to 5.0% (w/w), and more preferably 1.0 to 5.0% (w/w), based on the total weight of the composition. The skin care composition of the invention may also comprise more than one pH adjuster. In this case the above amounts preferably refer to the overall amount of pH adjusters in the composition.

In one preferred embodiment, the skin care composition of the present invention comprises a thickener. Thickeners are compounds that increase the viscosity of a cosmetic or pharmaceutical formulation. Thickeners are often polymers that absorb water and swell up, thereby making the composition more viscous. Suitable thickeners for the skin care composition of the present invention include, but are not limited to, bean gum, xanthan gum, gelatin, Carnauba wax, stearic acid, C. crispus extract, hydroxypropyl starch phosphate, and mixtures thereof. Preferably, the thickener is present in the skin care composition of the invention in an amount of 0.05% (w/w) to 10% (w/w), preferably 0.1 to 10.0% (w/w), and more preferably 1.0 to 10.0% (w/w), based on the total weight of the composition. In another embodiment, the thickener is present in the skin care composition of the invention in an amount of 0.05% (w/w) to 5.0% (w/w), preferably 0.1 to 5.0% (w/w), and more preferably 1.0 to 5.0% (w/w), based on the total weight of the composition. The skin care composition of the invention may also comprise more than one thickener. In this case the above amounts preferably refer to the overall amount of thickeners in the composition.

In one preferred embodiment, the skin care composition of the present invention comprises a solubilizer. As used herein, a solubilizer is a compound that aids in the solubilization of hydrophobic substances in aqueous and alcoholic formulations. For example, a solubilizer may render feasible the solubilization of perfume oils and other hydrophobic substances, such as vitamins, into an aqueous skin care composition. Suitable solubilizers for the skin care composition of the present invention include, but are not limited to, pentaerythrityl tetraisostearate, polyglyceryl-4 caprate, polyglyceryl-3 cocoate, polyglyceryl-10 caprylate and different poloxamers, such as poloxamer 188, 234, 235, 238 and 338. Preferably, the solubilizer is present in the skin care composition of the invention in an amount of 0.05% (w/w) to 10% (w/w), preferably 0.1 to 10.0% (w/w), and more preferably 1.0 to 10.0% (w/w), based on the total weight of the composition. In another embodiment, the solubilizer is present in the skin care composition of the invention in an amount of 0.05% (w/w) to 5.0% (w/w), preferably 0.1 to 5.0% (w/w), and more preferably 1.0 to 5.0% (w/w), based on the total weight of the composition. The skin care composition of the invention may also comprise more than one solubilizer. In this case the above amounts preferably refer to the overall amount of solubilizers in the composition.

In one preferred embodiment, the skin care composition of the present invention comprises an antioxidant. These compounds are normally added to cosmetic or pharmaceutical formulations to prevent oxidative reactions catalyzed by oxygen radicals that would otherwise result in the decomposition of ingredients in the composition, such as proteins, sugars, and lipids. Antioxidants which are commonly used in cosmetic product include chemicals like butylated hydroxytoluene, butylated hydroxyanisole, tocopherol, tocopheryl acetate and plant-derived polyphenols, flavonoids, flavanols, stilbens, and terpenes. Preferably, the antioxidant is present in the skin care composition of the invention in an amount of 0.05% (w/w) to 10% (w/w), preferably 0.1 to 10.0% (w/w), and more preferably 1.0 to 10.0% (w/w), based on the total weight of the composition. In another embodiment, the antioxidant is present in the skin care composition of the invention in an amount of 0.05% (w/w) to 5.0% (w/w), preferably 0.1 to 5.0% (w/w), and more preferably 1.0 to 5.0% (w/w), based on the total weight of the composition. The skin care composition of the invention may also comprise more than one antioxidant. In this case the above amounts preferably refer to the overall amount of antioxidants in the composition.

In one preferred embodiment, the skin care composition of the present invention comprises a preservative. As used herein, a preservative is a compound that is added to cosmetic or pharmaceutical formulation to prevent microbial spoilage of the formulation by inhibiting the growth of unintended bacteria and yeasts. Commonly used preservatives for cosmetic formulations include, amongst others, benzyl alcohol, ethanol, phenoxyethanol, caprylyl glycol, salicylic acid and sorbic acid. Preferably, the preservative is present in the skin care composition of the invention in an amount of 0.05% (w/w) to 10% (w/w), preferably 0.1 to 10.0% (w/w), and more preferably 1.0 to 10.0% (w/w), based on the total weight of the composition. In another embodiment, the preservative is present in the skin care composition of the invention in an amount of 0.05% (w/w) to 5.0% (w/w), preferably 0.1 to 5.0% (w/w), and more preferably 1.0 to 5.0% (w/w), based on the total weight of the composition. The skin care composition of the invention may also comprise more than one preservative. In this case the above amounts preferably refer to the overall amount of preservatives in the composition.

As used herein, all percentages are by weight of the skin care composition, unless specifically stated otherwise. All ratios are weight ratios, unless stated otherwise.

The novel strains of the present invention as well as culture supernatants obtained from any of these strains and skin care compositions comprising one or more of the strains or culture supernatants thereof are useful for the modulation of the skin microbiome, and in particular for reducing the number of *S. aureus* cells on the skin of a subject. The strains, culture supernatants and skin care compositions of the invention are therefore particularly useful for treating *S. aureus* skin infections and skin diseases which characterized by an excessive amount *of S. aureus* cells on the skin, such as atopic dermatitis and/or rosacea.

Thus, in one preferred aspect the invention relates to *S. epidermidis* strain HAC26 or S. *warneri* strain HAA333 or supernatants obtained from any of these strains or a combination of these strains or culture supernatants thereof or a skin care composition described hereinabove comprising one or more of these strains or culture supernatants thereof for use in a method of reducing the number of *S. aureus* cells on the skin of a subject. The invention also relates to the use of *S. epidermidis* strain HAC26 or *S. warneri* strain HAA333 or supernatants obtained from any of these strains or a combination of these strains or culture supernatants thereof or a skin care composition described hereinabove for reducing the number *of S. aureus* cells on the skin of a subject. The invention also relates to a method of reducing the number of *S. aureus* cells on the skin of a subject, said method comprising the administration of *S. epidermidis* strain HAC26 or *S. warneri* strain HAA333 or supernatants obtained from any of these strains or a combination of these strains or culture supernatants thereof or a skin care composition described hereinabove to the skin of a subject in need thereof. It is particularly preferred that the subject to be treated is a human.

In another preferred aspect, the invention relates to *S. epidermidis* strain HAC26 or *S. warneri* strain HAA333 or culture supernatants obtained from any of these strains or a combination of these strains or culture supernatants thereof or a skin care composition described hereinabove comprising one or more of these strains or culture supernatants thereof for use in a method of treating a *S*. *aureus* infection of the skin in a subject. The invention also relates to the use of *S. epidermidis* strain HAC26 or *S. warneri* strain HAA333 or supernatants obtained from any of these strains or a combination of these strains or culture supernatants thereof or a skin care composition described hereinabove for treating a *S*. *aureus* infection of the skin in a subject. The invention also relates to a method of treating a *S*. *aureus* infection of the skin in a subject, said method comprising the administration of *S. epidermidis* strain HAC26 or *S. warneri* strain HAA333 or supernatants obtained from any of these strains or a combination of these strains or culture supernatants thereof or a skin care composition described hereinabove to the skin of a subject in need thereof. It is particularly preferred that the subject to be treated is a human.

In yet another preferred aspect, the invention relates to *S. epidermidis* strain HAC26 or *S*. *warneri* strain HAA333 or supernatants obtained from any of these strains or a combination of these strains or culture supernatants thereof or a skin care composition described hereinabove comprising one or more of these strains or culture supernatants thereof for use in a method of treating atopic dermatitis and/or rosacea in a subject. The invention also relates to the use of *S. epidermidis* strain HAC26 or *S. warneri* strain HAA333 or supernatants obtained from any of these strains or a combination of these strains or culture supernatants thereof or a skin care composition described hereinabove for atopic dermatitis and/or rosacea in a subject. The invention also relates to a method of treating atopic dermatitis and/or rosacea in a subject, said method comprising the administration of *S. epidermidis* strain HAC26 or *S. warneri* strain HAA333 or supernatants obtained from any of these strains or a combination of these strains or culture supernatants thereof or a skin care composition described hereinaboveto the skin of a subject in need thereof. It is particularly preferred that the subject to be treated is a human.

The skin to be treated may include the skin of the face and the body, e.g. the skin of the neck, chest, back, arms, hands, legs or thighs. According to a preferred embodiment, the skin to be treated with the composition of the present invention is the skin of the face. According to another preferred embodiment, the skin to be treated with the composition of the present invention is the skin of the body.

The skin care compositions may be applied to the area of skin in need of treatment, e.g. the face or body, at least once a day, twice a day, or even more frequently if needed. When applied twice daily, the first and second applications are preferably separated by at least 6 hours, preferably 8 hours. Typically, the cosmetic composition is applied once in the morning and once in the evening. The composition of the invention can be used over long periods without any adverse side effects. For example, the period of treatment may be at least 1 week, at least 2 weeks, at least 3 weeks, at least 4 weeks, at least 6 weeks, at least 12 weeks, at least 24 weeks, or more. In some embodiments, the treatment will be extended for several months, such as for 4 months, 6 months, 8 months, 12 months, 18 months, or 24 months.

The skin care composition of the present invention may be provided as ready-to-use composition which allows for a direct topical administration to the skin. In such a composition, the lyophilized or spray-dried live bacteria will be present in admixture with other cosmetic or pharmaceutical excipients described elsewhere herein, such as emollients, fillers, and the like. Upon application of these compositions to the skin, the dried bacteria will be re-activated on the skin of the subject to which the product is applied. Growth of the re-activated bacteria from the skin care composition will positively influence the microbial flora on the skin of the subject.

These ready-to-use compositions are preferably stable at room temperature for at least 1 week, at least 2, weeks, at least 3 weeks, at least 4 weeks, at least 5 weeks, at least 6 weeks, at at least 8 weeks, at least 10 weeks, at least 12 weeks, at least 14 weeks, at least 16 weeks, at least 18 weeks, at least 20 weeks, at least 22 weeks, at least 24 weeks, at least 26 weeks, at least 28 weeks, or at least 30 weeks or more. As used herein, a composition is regarded as being stable if the reduction in the number of CFUs present in the composition after storage is less than a 3 log reduction, preferably less than a 2 log reduction, and more preferably less than a 1 log reduction. Stated differently, a composition is regarded as being stable if the reduction in the number of CFUs present in the composition after storage is less than 1000-fold, preferably less than 100-fold, and more preferably less 10-fold relative to the number of CFUs in the composition before storage.

The skin care compositions of the present invention may alternatively be provided as a kit-of-parts in which the lyophilized or spray-dried bacteria are spatially separated from the other components, e.g. the cosmetic or therapeutic components. For example, the kit-of-parts may be in the form of a packaging with two spatially separated chambers, wherein the first chamber contains the lyophilized or spray-dried bacteria, and the second chamber contains a cosmetic preparation, such as a water-containing cosmetic preparation. Prior to use, the contents of both chambers are mixed with each other, such as for example by a consumer or a patient, to provide a homogeneous skin care composition which is then applied to the skin. A kit-of-part assembly has the advantage that the bacteria can remain in lyophilized or spray-dried form until use which is associated with a particular high storage stability of the composition. In a kit-of-parts assembly, it is advantageous if the weight ratio of the bacteria in the first chamber to the cosmetic preparation, in particular the water-containing cosmetic preparation, in the second chamber is from 1:10 to 1:100, such as 1:10, 1:20, 1:30, 1:40, 1:50, 1:60, 1:70, 1:80, 1:90, or 1:100. After mixing the contents of both chambers, the skin care composition contains preferably 1-10% by weight lyophilized or spray-dried bacteria and 99-90% by weight of the cosmetic preparation, e.g. the water-containing cosmetic preparation. According to the present invention a kit-of-parts can be provided, for example, in a Lyo-Ject^{®} double-chamber syringe, in a V-LK^{®} double-chamber carpuel or in a dual-chamber system. In another preferred embodiment, the lyophilized or spray-dried bacteria in the first chamber may be suspended in a lipid or oil. This will significantly facilitate packaging and filling. In addition, the surrounding lipid or oil will protect the bacteria from premature rehydration. Preferably, the bacteria are suspended in ethylhexyl cocoate or dicaprylyl carbonate. The weight ratio of the bacteria to the oil or lipid preferably is between 1:1 and 1:2.

In one embodiment, the skin care composition of the present invention is an aqueous preparation, such as a gel. Aqueous preparations as intended herein encompass aqueous solutions, as well as aqueous dispersions. In one embodiment, the skin care composition is an oil-in-water emulsion. If the skin care composition contains an oil phase, e.g. when using an oil-in-water emulsion, it is preferred that the oil phase contains triglycerides and/or octyldodecanol. In addition, the oil phase may contain one or more oils selected from the group of lecithin, olive oil, sunflower oil, jojoba oil, soya oil, peanut oil, rapeseed oil, almond oil, palm oil, coconut oil, castor oil, wheat germ oil, grape seed oil, safflower oil, evening primrose oil, macadamia nut oil and the like.

The term "comprising", when used in the context with methods or compositions, means that other method steps or components of the composition can be present in addition to the method steps or components presented. The use of the term "comprising" indicates inclusion rather than limitation. For example, a composition "comprising" components A+B may also comprise C as a further component. Similarly, a method "comprising" steps (a) and (b) may also comprise (c) as a further method step. In contrast, the term "consisting of", when used in the context with methods or compositions, refers to methods or compositions which are exclusive of any other method steps or components of the composition not recited in the description of the respective composition or method. For example, a composition "consisting of" components A+B is limited to these two components and does not contain any other component apart from A and B. Similarly, a method "consisting of" steps (a) and (b) is a two-step method and does not contain any other method steps apart from (a) and (b). It should be understood, however, that any method or composition described herein as "comprising" certain method steps or components may preferably "consist essentially of' or may more preferably "consist of" the recited method steps or components. Further, unless otherwise required by context, singular terms shall include pluralities and plural terms shall include the singular.

### EXAMPLES

The following examples describe certain preferred embodiments of the present invention. It is however to be noted that the invention is not limited to such embodiments.

### Example 1: Sample acquisition and processing

Swab samples were collected from 30 volunteers (female, n=14; male, n=16) with an age range of 22-43 years from forehead, cheek, back and forearm skin, as described previously (Ahle et al., 2020. In brief, an area of 25 cm² of forehead, cheek, back skin and 50 cm² on forearm skin was swiped with a cotton swap which was pre-moistened in aqueous sampling buffer containing disodium phosphate (12.49 g/L, Merck), potassium dihydrogen phosphate (0.63 g/L, Merck) and 1 % Triton X-100 (Sigma). The swap was vigorously shaken in a tube containing 2 mL of sampling buffer and then removed. The sample was stored at -20°C before DNA extraction. Skin hydration and sebum content were measured with a Corneometer (Courage + Khazaka electronic) and Sebumeter (Courage + Khazaka electronic), respectively. None of the volunteers had a history of skin disease, nor had undergone treatment with topical medicine or antibiotics in the last six months. Written informed consent was obtained from all volunteers and the study was approved by International Medical & Dental Ethics Commission GmbH (IMDEC), Freiburg (Study no. 67885).

The swab samples were diluted (back, cheek, forehead skin sample: 1:10 and 1:1000; forehead skin sample: 1:1 and 1:100) in 0.9 % NaCl solution. Cultivation was done by plating on Columbia agar with 5 % sheep blood; agar plates were incubated at 37°C for 24 h. CFU count was determined with an automatic colony counter (IUL). Up to five colonies that resembled staphylococci based on colony size and color were randomly picked of each plate and pure cultures were obtained by sub-cultivation on the same agar. Each isolate of the 572 isolates that were obtained in total was assigned to species level by MALDI-TOF mass spectrometry.

Prior to DNA extraction, skin swab samples were centrifuged (8.000 g, 30 min at 4°C), and the supernatant was discarded. The pellets were lysed by using lysostaphin (0.05 mg/mL, Sigma) and lysozyme (9.5 mg/mL, Sigma). DNA was extracted using the DNeasy PowerSoil Kit (QIAGEN), following the manufacturer's instructions. DNA concentrations were measured with the Qubit dsDNA HS Assay (ThermoFisher Scientific) using a Qubit fluorometer.

Results: A total of 572 bacterial isolates were obtained via selective cultivation, of which 557 were identified as coagulase-negative staphylococci (CoNS) via MALDI-TOF mass spectrometry. Across all skin sites, the majority of isolates were identified as *S. epidermidis* (n=374, 67.2 %), followed by *Staphylococcus hominis* (n=86, 15.4 %). Forehead, cheek and back skin sites were dominated by strains of *S. epidermidis,* followed by *Staphylococcus capitis* (relative abundance of 74.5% and 11.7 %, respectively), whereas on forearm skin sites, a larger number of strains of *S. hominis* and *Staphylococcus haemolyticus* (relative abundance of 38.7 % and 7.3%, respectively) were isolated. Moisture content (skin hydration) was highest on back and forehead skin as compared to cheek and forearm skin (Fig. 1). The latter sites exhibited the highest sebum content and numbers of staphylococci per cm² (CFU), whereas forearm skin sites were particularly low in sebum and numbers of staphylococci (Fig. 2 and 3).

### Example 2: Antagonistic plate assay

All 557 CoNS isolates were screened for antimicrobial properties against the indicator strains *S. aureus DSM799* to identify staphylococcal isolates with bioactivity. Therefore, bacterial lawn plates were prepared. Liquid cultures of *S. aureus* were prepared in CASO broth. For staphylococci the liquid culture was adjusted to an optical density of OD600nm = 0.002. 6 mL of the adjusted culture was pipetted onto a rectangular Tryptic Soy Agar (TSA) plate and distributed evenly. For round TSA plates 3 mL bacterial suspension was used. After 30 sec excess liquid was removed and the plates were dried for 4 h. The plates were stored up to three weeks at 4°C.

The isolates were cultivated for 20 h at 37°C shaking in 1 mL CASO broth in 96-Deepwell plates. The 96-Deepwell plate was centrifuged at 2000 rpm for 5 min, 500 µL supernatant was removed and the pellet was re suspended in the remaining liquid. The concentrated bacterial cultures were transferred into 96-well U-bottom plates. Bacterial cultures and the supernatants were transferred on rectangular lawn plates with replicator stamps. After 4 h of drying, the plates were cultivated for 24 h at 37°C. A visible inhibition zone around a staphylococcal colony was regarded as antimicrobial activity. Staphylococcal strains that showed antimicrobial properties were verified in triplicates.

Results: It was observed 4 % (22/557) of the tested staphylococcal isolates exhibited activity against *S. aureus.* Among these 22 strains, *S. epidermidis* strain HAC26 and *S. warneri* strain HAA333 were found to have the highest antimicrobial activity against *S. aureus.* The results of the lawn plates experiments for the strains HAC26 and HAA333 are shown in Figure 4. In contrast to the control strain *S. epidermidis* ATCC12228, both test strains show a visible inhibition zone around the strain colony and the *S. aureus* cell lawn. In addition, inhibition of *S. aureus* can be observed when the supernatant of the test strains is used.

### Example 3: Preparing a skin care composition

The *S. epidermidis* strain HAC26 and the *S. warneri* strain HAA333 were incorporated into a skin care composition. For this purpose, a freeze-dried powder of the *S. epidermidis HAC26* and *S. warneri HAA333* was produced and mixed with other ingredients as indicated below. Ingredients are listed in % (w/w).

| INCI | % | |
|---|---|---|
| | 1 | 2 |
| Caprylic/Capric Triglyceride | 5 | 5 |
| Glycerin | 10 | 10 |
| Tocopherol | 0.15 | 0.15 |
| Hydrogenated Coco-Glycerides | 4 | 4 |
| Polyglyceryl-3 Diisostearate | 1.5 | 1.5 |
| Decyl Oleate | 17 | 17 |
| Octyldodecanol | 23.8 | 23.8 |
| Hydrogenated Castor Oil | 1 | 1 |
| Helianthus Annuus Seed Oil | 0.15 | 0.15 |
| Cera Alba | 1 | 1 |
| Ricinus Communis Seed Oil | 10 | 10 |
| Butyrospermum Parkii Butter | 8 | 8 |
| Cetyl Palmitate | 4 | 4 |
| Bis-Diglyceryl Polyacyladipate-2 | 4.7 | 4.7 |
| Dicaprylyl Carbonate | 5 | 5 |
| S. warneri HAA333 lyophilisate^{∗} | 4.7 | 0 |
| S. epidermidis HAC26 lyophilisate^{∗} | 0 | 4.7 |

| | | |
|---|---|---|
| ^{∗} CFU= 10¹⁰/g | | |

### LITERATURE

Ahle CM, et al. Staphylococcus saccharolyticus: An Overlooked Human Skin Colonizer, Microorganisms 2020, 8.
Brown SJ, Irvine AD. Atopic eczema and the filaggrin story. Semin Cutan Med Surg. 2008; 27(2): 128-137.
Dobie D & Gray J. Fusidic acid resistance in Staphylococcus aureus. Arch Dis Child 2004; 89: 74-77.
Miraglia del Giudice et al. Immune dysregulation in atopic dermatitis. Allergy Asthma Proc, 2006; 27(6): 451-5.
Ring J, et al. European Dermatology Forum (EDF); European Academy of Dermatology and Venereology (EADV); European Federation of Allergy (EFA); European Task Force on Atopic Dermatitis (ETFAD); European Society of Pediatric Dermatology (ESPD); Global Allergy and Asthma European Network (GA2LEN). Guidelines for treatment of atopic eczema (atopic dermatitis) part I. J Eur Acad Dermatol Venereol. 2012; 26(8):1045-1060.
Sandilands A et al. Filaggrin in the frontline: role in skin barrier function disease. J Cell Sci. 2009; 122(pt 9): 1285-1294.
Scharschmidt TC, et al. Filaggrin deficiency confers a paracellular barrier abnormality that reduces inflammatory thresholds to irritants and haptens. J Allergy Clin Immunol. 2009; 124(3): 496-506,506.e1-e6.
Sicherer SC, Leung DY. Advances in allergic skin disease, anaphylaxis, and hypersensitivity reactions to foods, drugs, and insects in 2008. J Allergy Clin Immunol. 2009; 123(2): 319-327.
Silverberg NB, Silverberg JI. Inside out or outside in: does atopic dermatitis disrupt barrier function or does disruption of barrier function trigger atopic dermatitis? Cutis. 2015;96(6):359-361.

## Claims

1. Skin care composition for topical administration to the skin comprising
(a) *Staphylococcus epidermidis* strain HAC26, deposited at the DSMZ under accession number DSM 34117,
(b) *Staphylococcus warneri* strain HAA333, deposited at the DSMZ under accession number DSM 34118, and/or
(c) culture supernatant obtained from any of the strains recited in (a)-(b).

2. Skin care composition of claim 1, wherein said *Staphylococcus epidermidis* strain is present in the composition in lyophilized or spray-dried form.

3. Skin care composition of claim 1 or 2, wherein said *Staphylococcus epidermidis* strain is present in the composition in an amount of
(a) 10⁴-10¹¹ CFU/ml, preferably 10⁷-10¹⁰ CFU/ml of the skin care composition, and/or
(b) at least 0.5% (w/v) of the skin care composition.

4. Skin care composition of any of claims 1-3, wherein said *Staphylococcus warneri* strain is present in the composition in lyophilized or spray-dried form.

5. Skin care composition of claim 1 or 3, wherein said *Staphylococcus warneri* strain is present in the composition in an amount of
(a) 10⁴-10¹¹ CFU/ml, preferably 10⁷-10¹⁰ CFU/ml, or
(b) at least 0.5% (w/v) of the skin care composition.

6. Skin care composition of any of claims 1-5, wherein said composition comprises
(a) *Staphylococcus epidermidis* strain HAC26 in an amount of 10⁴-10¹¹ CFU/ml, and
(b) *Staphylococcus warneri* strain HAA333 in an amount of 10⁴-10¹¹ CFU/ml.

7. Skin care composition of any of claims 1-6, wherein said composition further comprises one or more excipients.

8. Skin care composition of claim 7, wherein said one or more excipients comprise an emollient, a filler, a thickener, a solubilizer, an antioxidant, a preservative, a pH adjuster, a binder, a buffering agent, a colorant, a humectants, a exfoliating agent, a preservative, a plant extract, an essential oils, or a fragrance.

9. Skin care composition of any of claims 1-8, wherein said composition is in the form of a gel, cream, ointment or lotion.

10. *Staphylococcus epidermidis* strain HAC26, deposited at the DSMZ under accession number DSM 34117 or culture supernatant obtained from said strain.

11. *Staphylococcus warneri* strain HAA333, deposited at the DSMZ under accession number DSM 34118 or culture supernatant obtained from said strain.

12. Skin care composition of any of claims 1-9 or bacterial strain or culture supernatant of any of claims 10-11 for use in a method of treating atopic dermatitis and/or rosacea in a subject.

13. Skin care composition of any of claims 1-19 or bacterial strain or culture supernatant of any of claims 10-11 for use in a method of reducing the number of *Staphylococcus aureus* cells on the skin of a subject.

14. Skin care composition of any of claims 1-19 or bacterial strain or culture supernatant of any of claims 10-11 for use in a method of treating an infection of the skin of a subj ect.
